# EUROPEAN PATENT APPLICATION

(11) **EP 1 284 290 A1**
(43) Date of publication of application: **19.02.2003**
(21) Application number: 01118775.4
(22) Date of filing: 08.08.2001
(51) Int. Cl.: C12N 15/12, C12N 15/67, C07K 14/755

(54) **Increase of the expression levels of factor VIII by insertion of spliceable nucleotide sequences into factor VIII cDNA**

(71) Applicant: Aventis Behring GmbH, 35002 Marburg (DE)
(72) Inventor: Negrier, Claude, Prof. Dr., 69540 Irigny (FR); Plantier, Jean-Luc, Dr., 69520 Gringy (FR); Rodriguez, Marie-Hélène, Dr., 38080 L'isle d'Abeau (FR); Hauser, Hans-Peter, Dr., 35041 Marburg (DE)

(57) **Abstract**

A modified factor VIII cDNA is disclosed, wherein in introns 1 and/or 13 of the wild-type factor VIII cDNA one or more splicable nucleotide sequences or a nucleotide sequence which will be spliced during the export of the pre-mRNA from the nucleus have been inserted. Further a process for the production of a biologically active recombinant human factor VIII or its derivative is disclosed, which is performed by cultivating an animal cell line comprising a recombinant expression vector containing said modified factor VIII cDNA. Moreover a transfer vector for use in the human gene therapy is described which comprises said modified factor VIII cDNA.

## Description

The present invention relates to modified DNA sequences coding for biologically active recombinant human factor VIII and its derivatives, recombinant expression vectors containing such DNA sequences, host cells transformed with such recombinant expression vectors, and processes for the manufacture of the recombinant human factor VIII and its derivatives. The invention also covers a transfer vector for use in human gene therapy which comprises such modified DNA sequences.

Classic hemophilia or hemophilia A is the most common of the inherited bleeding disorders. It results from a chromosome X-linked deficiency of blood coagulation factor VIII, and affects almost exclusively males with an incidence of between one and two individuals per 10.000. The X-chromosome defect is transmitted by female carriers who are not themselves hemophiliacs. The clinical manifestation of hemophilia A is an abnormal bleeding tendency and before treatment with factor VIII concentrates was introduced the mean life span for a person with severe hemophilia was less than 20 years. The use of concentrates of factor VIII from plasma has considerably improved the situation for the hemophilia patients. The mean life span has increased extensively, giving most of them the possibility to live a more or less normal life. However, there have been certain problems with the plasma derived concentrates and their use, the most serious of which have been the transmission of viruses. So far, viruses causing AIDS, hepatitis B, and non A non B hepatitis have hit the population seriously. Although different virus inactivation methods and new highly purified factor VIII concentrates have recently been developed no guarantees on the absence of virus contamination can be made. Also, the factor VIII concentrates are fairly expensive because the limited supply of human plasma raw material.

A factor VIII product derived from recombinant material is likely to solve a large extent of the problems associated with the use of plasma derived factor VIII concentrates for treatment for hemophilia A. However, the development of a recombinant factor VIII has met with some difficulties, for instance the problem of achieving production levels in sufficiently high yields, in particular regarding the full-length molecule.

In fresh plasma prepared in the presence of protease inhibitors, factor VIII has been shown to have a molecular weight of 280 kDa and to be composed of two polypeptide chains of 200 kDa and 80 kDa, respectively (Andersson, L.-O., et al. (1986) Proc. Natl. Aca. Sci. USA 83, 2979-2983). These chains are held together by metal ion bridges. More or less proteolytically degraded forms of the factor VIII molecule can be found as active fragments in factor VIII material purified from commercial concentrates (Andersson, L.-O., et al. ibid.; Andersson, L.-O., et al. (1985) EP 0 197 901). The fragmented form of factor VIII having molecular weights from 260 kDa down to 170 kDa, consists of one heavy chain with a molecular weight ranging from 180 kDa down to 90 kDa, where all variants have identical amino termini, in combination with one 80 kDa light chain. The amino-terminal region of the heavy chain is identical to that of the single chain factor VIII polypeptide that can be deduced from the nucleotide sequence data of the factor VIII cDNA (Wood, W.I., et al. (1984) Nature 312, 330-336; Vehar, G.A., et al. (1984) Nature 312, 337-342).

The smallest active form of factor VIII with a molecular weight of 170 kDa, consisting of one 90 kDa and one 80 kDa chain, can be activated with thrombin to the same extent as the higher molecular weight forms, and thus represents an unactivated form. It has also been shown to have full biological activity in vivo as tested in hemophilia dogs (Brinkhous, K.M., et al. (1985) Proc.Natl.Acad.Scl. USA 82, 8752-8756). Thus, the haemostatic effectiveness of the 170 kDa form is the same as for the high molecular weight forms of factor VIII.

The fact that the middle heavily glycosylated region of the factor VIII polypeptide chain residing between amino acids Arg-740 and Glu-1649 does not seem to be necessary for full biological activity has prompted several researchers to attempt to produce derivatives of recombinant factor VIII lacking this region. This has been achieved by deleting a portion of the cDNA encoding the middle heavily glycosylated region of factor VIII either entirely or partially.

For example, J.J. Toole, et al, reported the construction and expression of factor VIII lacking amino acids 982 through 1562, and 760 through 1639 respectively (Proc.Natl.Acad.Scl. USA (1986) 83, 5939-5942). D.L. Eaton, et al. reported the construction and expression of factor VIII lacking amino acids 797 through 1562 (Biochemistry (1986) 25, 8343-8347). R.J. Kaufman described the expression of factor VIII lacking amino acids 741 through 1646 (PCT application No. WO 87/04187). N. Sarver, et al. reported the construction and expression of factor VIII lacking amino acids 747 through 1560 (DNA (1987) 6, 553-564). M. Pasek reported the construction and expression of factor VIII lacking amino acids 745 through 1562, and amino acids 741 through 1648, respectively (PCT application No. WO 88/00831). K.-D. Langner reported the construction and expression of factor VIII lacking amino acids 816 through 1598, and amino acids 741 through 1689, respectively (Behring Inst. Mitt., (1988) No. 82, 16-25, EP 295 597). P. Meulien, et al., reported the construction and expression of factor VIII lacking amino acids 868 through 1562, and amino acids 771 through 1666, respectively (Protein Engineering (1988) 2(4), 301-306, EP 0 303 540 A1). When expressing these deleted forms of factor VIII cDNA in mammalian cells the production level is typically 10 times higher as compared to full-length factor VIII.

Furthermore, attempts have been made to express the 90 kDa and 80 kDa chains separately from two different cDNA derivatives in the same cell (Burke, R.L., et al. (1986), J. Biol. Chem. 261, 12574-12578, Pavirani, A., et al. (1987) Biochem. Biophys. Res. Comm., 145, 234-240). However, in this system the in vivo reconstitution seems to be of limited efficiency in terms of recovered factor VIII; C activity.

Several studies have stressed the low FVIII production level in different cellular systems: Biosynthesis of FVIII was shown to be regulated in at least three different levels. First, among the FVIII cDNA sequence two nucleotides stretches, localized in the A2 coding domain, were demonstrated to act as transcriptional silencers (Fallaux et al., 1996; Hoeben et al., 1995; Koeberl et al., 1995; Lynch et al., 1993): Second, FVIII protein synthesis is tightly regulated by several reticulum endoplasmic chaperones (BIP; Calreticulin; Calnexin; ERGIC-53). Many of these interactions retain FVIII in the cell and direct it through the cellular degradation machinery (Dorner et al., 1987; Nochols et al., 1998; Pipe et al., 1998). Third, once secreted FVIII is sensitive to protease degradation and needs to be protected by von Willebrand Factor (vWF) (Kaufman et al., 1989).

It is therefore a problem to develop improved processes which result in higher yields of FVIII. The present invention offers a solution to this problem by a modified FVIII cDNA.

This invention describes deleted factor VIII cDNA molecules that code for recombinant factor VIII derivatives, corresponding, with regard to molecular weight and other biochemical characteristics, to a previously derived plasma factor VIII form present in considerable amounts in commercial concentrates (Andersson, L.-O. et al., (1986), Proc. Natl. Acad. Scl. USA 83, 2979-2983). These new factor VIII cDNA derivatives give sufficiently high yields of recombinant factor VIII protein to be used in an industrial process for a pharmaceutical preparation of recombinant factor VIII or its derivatives.

This invention is based on the observation that the lack of introns 1 and/or 13 of the wild-type factor VIII genomic DNA in the cDNA of factor VIII prevents high level expression of factor VIII and therefore the cDNA needs to be modified. This can effectively be done by the insertion of a truncated FIX intron in the position of intron 1 and/or 13 as described in European patent application 1 048 726. However it was surprisingly found that this is not restricted to a truncated FIX intron but that the introduction of one or more splicable nucleotide sequences or one or more nucleotide sequences which will be spliced during the export of the pre-mRNA from the nucleus into the position of introns 1 and/or 13 of the wild-type factor VIII genomic DNA. A preferred embodiment of the invention consists in a modified factor VIII cDNA wherein at the positions of introns 1 and/or 13 of the wild-type factor VIII genomic DNA one or more complete or truncated introns or one or more synthetic introns which retain the ability to be spliced have been inserted. Excellent results have been obtained by the insertion of the first complete or truncated Apolipoprotein A1 intron or the first complete or truncated β-Globin intron at the position of introns 1 and/or 13 of the wild-type factor VIII genomic DNA.

A further object of this invention is to improve the level of expression of factor VIII and its derivatives by use of a modified factor VIII cDNA in which the B-domain of the wild-type factor VIII cDNA has been shortened or completely eliminated.

Preferably a modified factor VIII cDNA is used which comprises a first DNA segment coding for the amino acids 1 through 740 of the human factor VIII and a second DNA segment coding for the amino acids 1649 through 2332 of the human factor VIII. These two segments may be interconnected by a linker DNA segment preferably coding for a linker peptide of at least two amino acids which are selected from lysine or arginine as described in International patent application WO 92/16557.

According to the present invention such B-domain deleted factor VIII cDNA may be further modified in that at the position of intron 1 and/or 13 of the wild type genomic DNA of factor VIII one or more splicable nucleotide sequences or a nucleotide sequence which will be spliced during the export of the pre-mRNA from the nucleus are inserted.

Especially, if at the position of introns 1 and/or 13 of wild-type genomic DNA of factor VIII one or more complete or truncated introns or one or more synthetic introns which retain the ability to be spliced are inserted the level of expression of factor VIII is considerably increased. Examples of well suited introns for insertion into the position of intron 1 and/or 13 of wild-type genomic DNA of factor VIII are the first complete or truncated Apolipoprotein A1 intron or the first complete or truncated β-Globin intron.

The production of factor VIII proteins at high levels in suitable host cells, requires the assembly of the above-mentioned modified factor VIII DNA's into efficient transcriptional units together with suitable regulatory elements in a recombinant expression vector, that can be propagated in E. coli according to methods known to those skilled in the art. Efficient transcriptional regulatory elements could be derived from viruses having animal cells as their natural hosts or from the chromosomal DNA of animal cells. Preferably, promoter-enhancer combinations derived from the Simian Virus 40, adenovirus, BK polyoma virus, human cytomegalovirus, or the long terminal repeat of Rous sarcoma virus, or promoter-enhancer combinations including strongly constitutively transcribed genes in animal cells like beta-actin or GRP78 can be used. In order to achieve stable high levels of mRNA transcribed from the factor VIII DNA's, the transcriptional unit should contain in its 3'-proximal part a DNA region encoding a transcriptional termination-polyadenylation sequence. Preferably, this sequence is derived from the Simian Virus 40 early transcriptional region, the rabbit beta-globin gene, or the human tissue plasminogen activator gene.

The factor VIII cDNA's thus assembled into efficient recombinant expression vector are then introduced into a suitable host cell line for expression of the factor VIII proteins. Preferably this cell line should be an animal cell-line of vertebrate origin in order to ensure correct folding, disulfide bond formation, asparagines-linked glycosylation and other post-translational modifications as well as secretion into the cultivation medium. Examples on other post-translational modifications are tyrosine O-sulfation, and proteolytic processing of the nascent polypeptide chain. Examples of cell lines that can be use are monkey COS-cells, mouse L-cells, mouse C127-cells, hamster BHK-21 cells, human embryonic kidney 293 cells, and preferentially CHO-cells.

The recombinant expression vector encoding the factor VIII cDNA's can be introduced into an animal cell line in several different ways. For instance, recombinant expression vectors can be created from vectors based on different animal viruses, Examples of these are vectors based on baculovirus, vaccinia virus, adenovirus, and preferably bovine papilloma virus.

The transcription units encoding the factor VIII DNA's can also be introduced into animal cells together with another recombinant gene which may function as a dominant selectable marker in these cells in order to facilitate the isolation of specific cell clones which have integrated the recombinant DNA into their genome. Examples of this type dominant selectable marker genes are Tn5 aminoglycoside phosphotransferase, conferring resistance to Geneticin (G418), hygromycin phosphotransferase, conferring resistance to hygromycin, and puromycin acetyl transferase, conferring resistance to puromycin. The recombinant expression vector encoding such a selectable marker can reside either on the same vector as the one encoding the factor VIII cDNA, or it can be encoded on a separate vector which is simultaneously introduced and integrated to the genome of the host cell, frequently resulting in a tight physical linkage between the different transcription units.

Other types of selectable marker genes which can be used together with the factor VIII DNA's are based on various transcription units encoding dihydrofolate reductase (dhfr). After introduction of this type of gene into cells lacking endogenous dhfr-activity, preferentially CHO-cells (DUKX-B11, DG-44) it will enable these to grow in media lacking nucleosides. An example of such a medium is Ham's F12 without hypoxanthin, thymidin, and glycine. These dhfr-genes can be introduced together with the factor VIII cDNA transcriptional units into CHO-cells of the above type, either linked on the same vector or on different vectors, thus creating dhfr-positive cell lines producing recombinant factor VIII protein.

If the above cell lines are grown in the presence of the cytotoxic dhfr-inhibitor methotrexate, new cell lines resistant to methotrexate will emerge. These cell lines may produce recombinant factor VIII protein at an increased rate due to the amplified number of linked dhfr and factor VIII transcriptional units. When propagating these cell lines in increasing concentrations of methotrexate (1-10000 nM), new cell lines can be obtained which produce factor VIII protein at very high rate.

The above cell lines producing factor VIII protein can be grown on a large scale, either in suspension culture or on various solid supports. Examples of these supports are microcarriers based on dextran or collagen matrices, or solid supports in the form of hollow fibres or various ceramic materials. When grown in suspension culture or on microcarriers the culture of the above cell lines can be performed either as a bath culture or as a perfusion culture with continuous production of conditioned medium over extended periods of time. Thus, according to the present invention, the above cell lines are well suited for the development of an industrial process for the production of recombinant factor VIII that can be isolated from human plasma.

The recombinant factor VIII protein which accumulate in the medium of CHO-cells of the above type, can be concentrated and purified by a variety of biochemical methods, including methods utilizing differences in size, charge, hydrophobicity, solubility, specific affinity, etc. between the recombinant factor VIII protein and other substances in the cell cultivation medium.

An example of such a purification is the adsorption of the recombinant factor VIII protein to a monoclonal antibody which is immobilised on a solid support. After desorption, the factor VIII protein can be further purified by a variety of chromatographic techniques based on the above properties.
The recombinant proteins with factor VIII activity described in this invention can be formulated into pharmaceutical preparations for therapeutic use. The purified factor VIII proteins may be dissolved in conventional physiologically compatible aqueous buffer solutions to which there may be added, optionally, pharmaceutical adjuvants to provide pharmaceutical preparations.

The modified factor VIII DNA's of this invention may also be integrated into a transfer vector for use in the human gene therapy.

The present invention will be further described more in detail in the following examples thereof. This description of specific embodiments of the invention will be made in conjunction with the appended drawings.

### Preparation of the FVIII A1 + 13 and FVIII B1+13 constructs

### Cloning of the introns

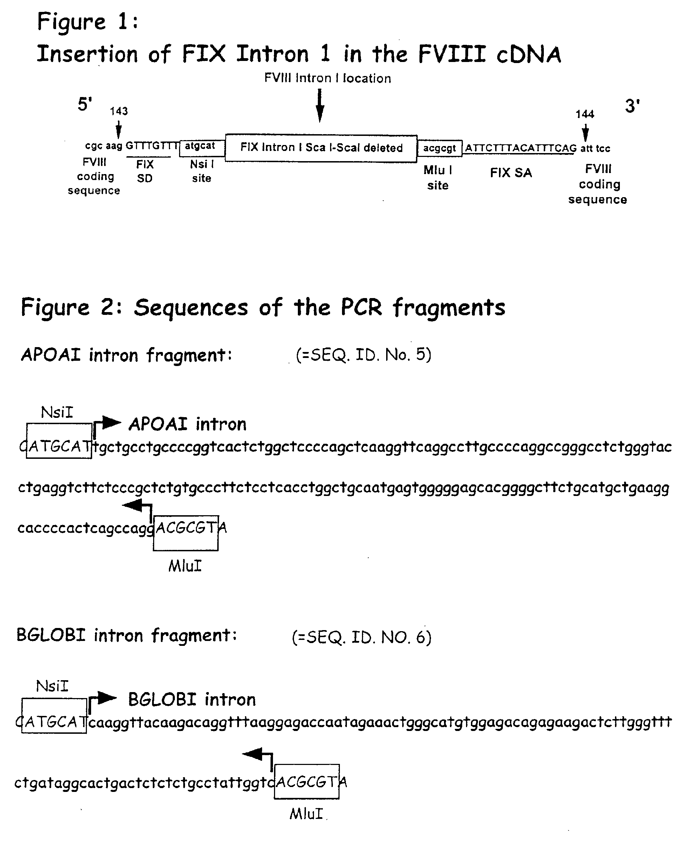

To clone the first introns of the apolipoproteine A1 (A) and the β-globin (B) genes in place of the FIX intron 1, two set of primers were designed. The new intronic sequences inserted between the splice donor (SD) and the splice acceptor (SA) of the FIX intron 1 are shown in Fig. 1. The two sets of oligonucleotides amplified the intron deleted of the respective SD and SA sites (5' cloning site, Nsil and 3' cloning site, Mlul). The following primers were used:

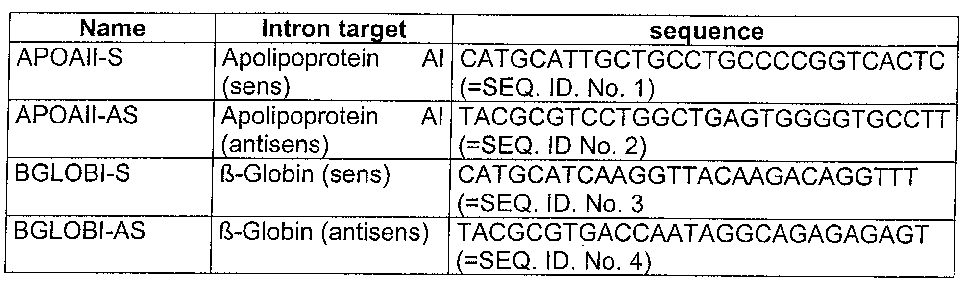

PCR reactions were performed using genomic DNA. The apolipoprotein A1 intron I (APOAI intron) gave a 186 base pairs (bp) length fragment and the β-globulin intron
I (BGLOBI intron) gave a 119 bp length fragment (Fig. 2). OCR fragments were cloned using TOPO TA cloning kit (pCR II vector: Invitrogen, Leek, the Netherlands).

### Insertion of each intron in position 1 or 13 in the FVIII cDNA's

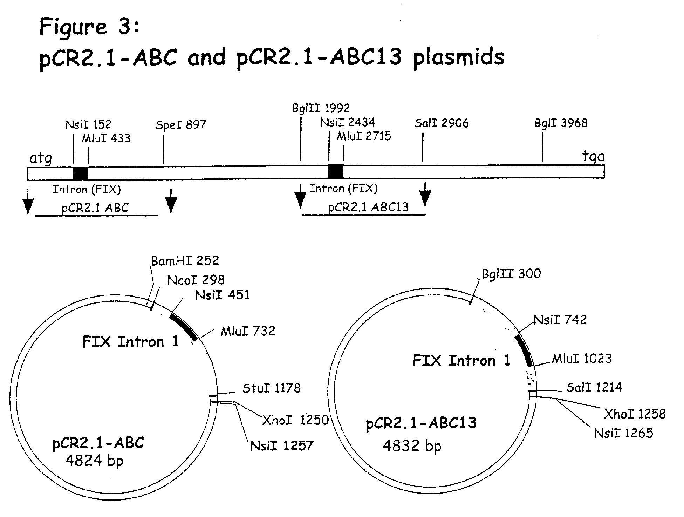

To insert the introns in the FVIII cDNA 2 plasmids were used which were obtained during the initial cloning of the FIX intron 1 (Fig. 3). pCR2.1 ABC comprises the FVIII ATG fragment (Ncol-Spel) with the FIX intron in position 1. pCR2.1 ABC13 contains a BgIII-SaII fragment of the FVIII cDNA with the FIX intron in position 13.

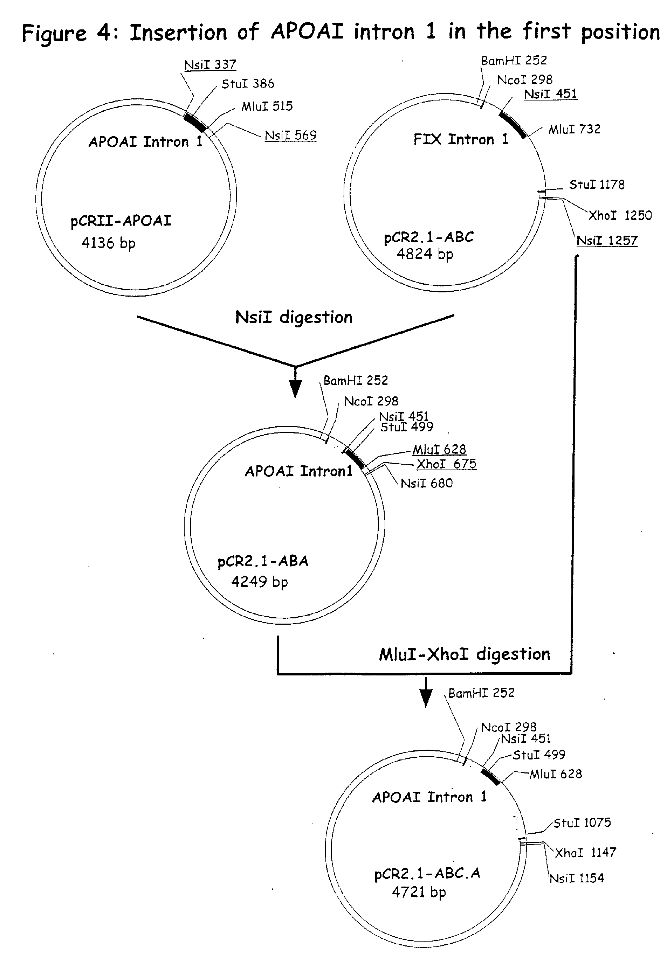

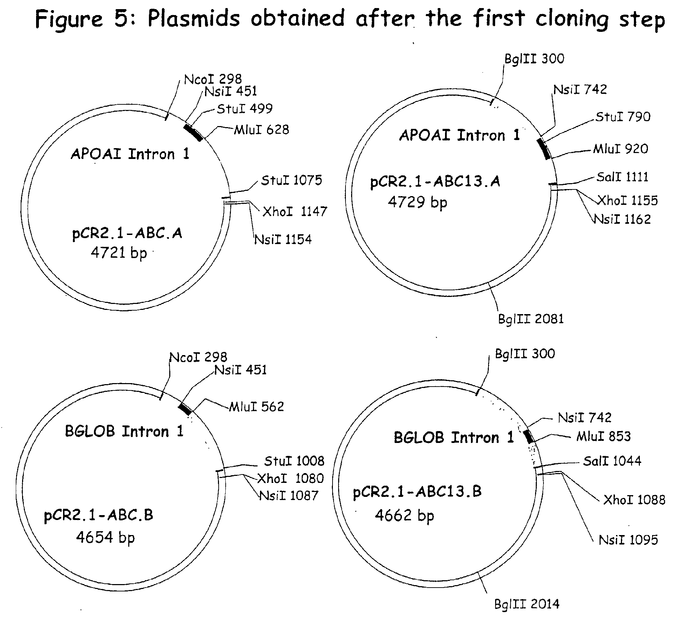

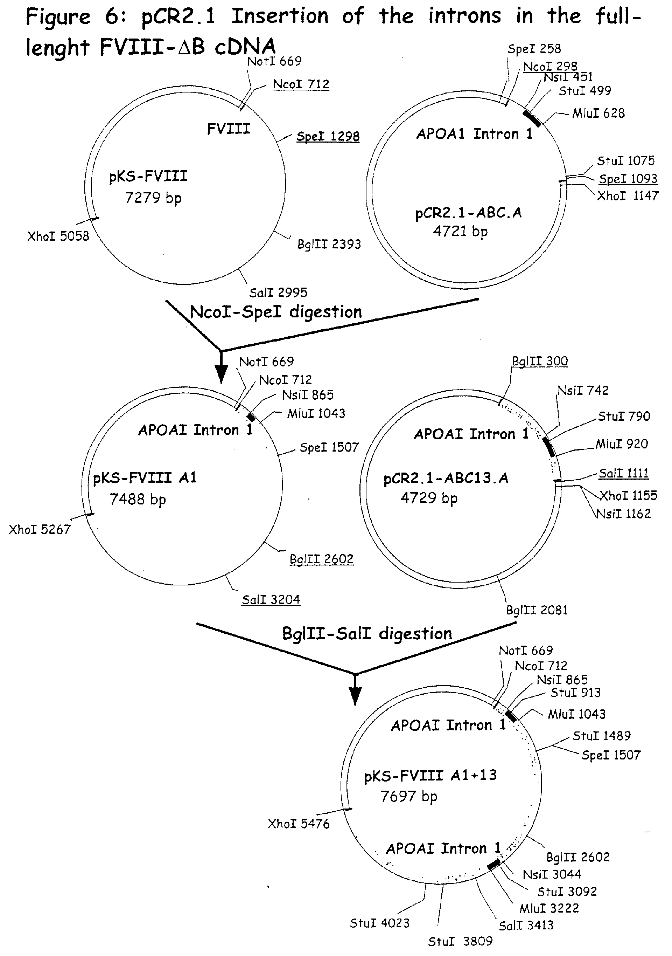

The APOAI intron was inserted in the pCR2.1 ABC using a Nsil digestion (Fig. 4). The Mlul-Xhol fragment of the pCR2.1 ABC was thereafter re-introduced in the obtained vector. The final plasmid was pCR2.1 ABC.A comprising the ATG fragment of the FVIII with APOAI intron in position 1. The same strategy was used to clone the BGLOB intron in position 1. The same Nsil ligation followed by the reintroduction of the Mlul-Xhol fragment was also used for the position 13. The obtained vectors were presented in Fig. 5.

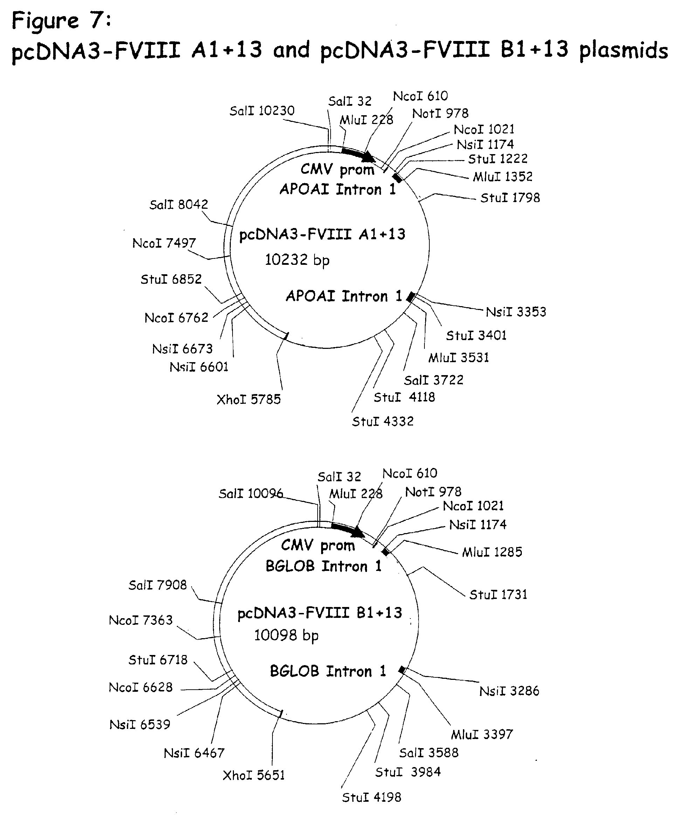

Spel. After ligation pKS-FVIII A1 was obtained. This vector comprised the APOAI intron in position 1 and was subsequently digested with BgIII-SaII digestion in order to introduce the intron in position 13. The final plasmid was pKS-FVIII A1 + 13 exhibiting the 2 intronic sequences. The same strategy was used to obtain pKS-FVIII B1 + 13. These two plasmids were subsequently digested by Notl and Xhol and the inserts were ligated in pcDNA3 vector (Invitrogen, Leek, the Netherlands) opened by the same enymes. The final expression plasmids were called pcDNA3-FVIII A1 + 13 and pcDNA3 FVIII B1 + 13 (Fig. 7).

### References:

Gallo-Penn AM., Shirley PS, Andrews JL, Kayda DB, Pinkstaff AM, Kaloss M, Tinlin S, Cameron C, Notley C, Hough C, Lilicrop D, Kaleko M, Conelly S (1999). In vivo evaluation of an adenoviral vector encoding canine factor VIII: high-level, sustained expression in hemophiliac mice. Hum. Gene Ther., 10(11), 1791-1802.

## Claims

1. Modified factor VIII cDNA, **characterized in that** in introns 1 and/or 13 of the wild-type factor VIII cDNA one or more splicable nucleotide sequences or a nucleotide sequence which will be spliced during the export of the pre-mRNA from the nucleus have been inserted.

2. Modified factor VIII cDNA as claimed in claim 1, **characterized in that** in introns 1 and/or 13 of the wild type factor VIII cDNA one or more complete or truncated introns have been inserted.

3. Modified factor VIII cDNA as claimed in claim 1, **characterized in that** in introns 1 and/or 13 of the wild-type factor VIII cDNA one or more synthetic introns which retain the ability to be spliced have been inserted.

4. Modified factor VIII cDNA as claimed in claim 1, **characterized in that** in introns 1 and/or 13 of the wild type factor VIII cDNA the first complete or truncated Apolipoprotein Al intron or the first complete or truncated β-Globulin intron have been inserted.

5. Modified factor VIII cDNA as claimed in claims 1 or 4, **characterized in that** it comprises a first DNA segment coding for the amino acids 1 through 740 of the human factor VIII and a second DNA segment coding for the amino acids 1649 through 2332 of the human factor VIII, said segments being interconnected by a

6. Recombinant expression vector containing a transcription unit comprising the modified factor VIII cDNA sequence according to claims 1 to 5, a transcriptional promoter and a polyadenylation sequence.

7. A host cell line of animal origin transformed with the recombinant expression vector of claim 6.

8. Process for the production of a biologically active recombinant human factor VIII or its derivative, **characterized in that** the production is performed by cultivating the animal cell line of claim 7 in a nutrient medium allowing expression and secretion of the human factor VIII or its derivative and recovering said expression product from the culture medium.

9. The human factor VIII or its derivative whenever prepared by the process of claim 8.

10. Pharmaceutical composition containing FVIII as described in claim 9.

11. Transfer vector for use in the human gene therapy, **characterized in that** it comprises a modified factor VIII cDNA as claimed in claims 1 to 5.


SEQUENCE LISTING

<110> Aventis Behring GmbH

<120> Modified cDNA for high Expression Levels of Factor VIII and its Derivatives

<130> C9P62EP

<140>
<141>

<160> 6

<170> PatentIn Ver. 2.1

<210> 1
<211> 28
<212> DNA
<213> Homo sapiens

<400> 1
catgcattgc tgcctgcccc ggtcactc 28

<210> 2
<211> 28
<212> DNA
<213> Homo sapiens

<400> 2
tacgcgtcct ggctgagtgg ggtgcctt                                                28

<210> 3
<211> 27
<212> DNA
<213> Homo sapiens

<400> 3
catgcatcaa ggttacaaga caggttt                                                 27

<210> 4
<211> 27
<212> DNA
<213> Homo sapiens

<400> 4
tacgcgtgac caataggcag agagagt                                                 27

<210> 5
<211> 186
<212> DNA
<213> Homo sapiens

<400> 5
catgcattgc tgcctgcccc ggtcactctg gctccccagc tcaaggttca ggccttgccc 60
caggccgggc ctctgggtac ctgaggtctt ctcccgctct gtgcccttct cctcacctgg 120
ctgcaatgag tgggggagca cggggcttct gcatgctgaa ggcaccccac tcagccagga 180
cgcgta 186

<210> 6
<211> 119
<212> DNA
<213> Homo sapiens

<400> 6
catgcatcaa ggttacaaga caggtttaag gagaccaata gaaactgggc atgtggagac 60
agagaagact cttgggtttc tgataggcac tgactctctc tgcctattgg tcacgcgta 119